# EUROPÄISCHE PATENTSCHRIFT

(11) **EP 0 871 913 B1**
(45) Veröffentlichungstag und Bekanntmachung des Hinweises auf die Patenterteilung: **07.08.2002**
(21) Anmeldenummer: 96916065.4
(22) Anmeldetag: 14.05.1996
(51) Int. Cl.: G02B 21/22, G02B 21/00, A61B 19/00

(54) **MIKROSKOP**
MICROSCOPE
MICROSCOPE

(30) Priorität: 17.05.1995 CH 144295
(43) Veröffentlichungstag der Anmeldung: 21.10.1998
(73) Patentinhaber: Leica Mikroskopie Systeme AG, 9435 Heerbrugg (CH)
(72) Erfinder: SPINK, Roger, CH-9442 Berneck (CH)
(74) Vertreter: Stamer, Harald, Dipl.-Phys.
(86) Internationale Anmeldenummer: EP9602057
(87) Internationale Veröffentlichungsnummer: WO9636897

(56) Entgegenhaltungen:
- EP-A- 0 367 246
- EP-A- 0 473 343
- EP-A- 0 562 742
- EP-A- 0 629 963
- US-A- 4 722 056

## Beschreibung

Die Erfindung betrifft ein Mikroskop mit wenigstens einem Strahlengang und einer Optik entlang einer optischen Achse und mit einem Einblendelement für das Einspiegeln von Bildinformationen für ein Betrachterauge. Unter Mikroskopen im Sinne der Erfindung sind vor allem - jedoch nicht ausschliesslich - Einrichtungen zu verstehen, die ein Hauptobjektiv, einen Tubus und ein Okular zum Hineinschauen aufweisen. Im weitesten Verständnis der Erfindung sind damit auch alle anderen optisch vergrössernden Einrichtungen zu verstehen, die auf ein zu vergrösserndes Objekt gerichtet werden und dem Betrachterauge ein vergrössertes Bild des betrachteten Objektes sichtbar machen. Mikroskope, insbesondere Stereomikroskope, beispielsweise Operationsmikroskope, insbesondere auch Video(-stereo-)mikroskope, die mit einer elektronischen Datenverarbeitungseinheit und/oder einem Display verbunden sind, sind dann für einen Anwender komfortabel, wenn dieser nicht ausschliesslich auf das aktuell durch das Hauptobjektiv des Mikroskopes gesehene Bild angewiesen ist, sondern beim Blick in den Tubus des Mikroskopes noch zusätzliche Informationen erhält, die dem aktuell gesehenen Bild in der Regel überlagert sind. Das können Schriftzeichen, Symboldarstellungen und Markierungen, aber auch überlagerte Bilder vom selben Objekt sein, die z.B. mit Hilfe einer Bildverarbeitungssoftware vom aktuell gesehenen Objekt oder durch andere Visualisierungsmassnahmen (z.B. Röntgenaufzeichnungen, CT usw.) vom selben Objekt gewonnen werden.

Mikroskope mit Einblend- bzw. Bild-Überlagerungsmöglichkeiten werden unter anderem auch in der Technik - z.B. Werkstofftechnik, Materialanalyse, Siliziumtechnik, Kriminologie usw. -, insbesondere jedoch auch in der Medizin für Diagnose, serologische Untersuchungen, bei Operationen usw. angewendet.

Vor allem bei Operationsmikroskopen und insbesondere während einer Operation fallen eine Menge von Informationen an, die für den Chirurgen von grosser Bedeutung sein können. Dies sind beispielsweise Informationen über den Patienten bzw. dessen Gesundheitszustand bzw. dessen Parameter wie Puls, Blutdruck, Sauerstoffgehalt des Blutes usw. Dies sind zusätzlich zu den aktuell betrachteten überlagernden Bildern z.B. Informationen über bestimmte Parameter des Mikroskops, Informationen über die Lage des beobachteten Operationsfeldes ebenso wie Steuerdaten, die beispielsweise vom Chirurgen willkürlich über Steuerorgane wie Computermouse oder Fussschalter an die Datenverarbeitung bzw. an Steuerorgane für das Mikroskop abgegeben werden, um dieses nach Bedarf zu steuern, z.B. zu fokussieren usw.

In einer Anordnung gemäß US-A-4 722 056 wird eine Bildinformation über eine Kathodenstrahlröhre (CRT) abgebildet und in den Strahlengang eines Mikroskopes eingespiegelt. Bedingt durch die physikalischen Eigenschaften einer Kathodenstrahlröhre sind die Möglichkeiten hinsichtlich Auflösung, Helligkeit und Randschärfe beschränkt.

Im folgenden wird beispielhaft insbesondere auf die Verwendung der Erfindung im Bereich der Operationsmikroskopie eingegangen. Deren Anwendung in den übrigen Bereichen fällt jedoch ebenso unter den Schutzbereich der Patentansprüche.

Operationsmikroskope dienen dem Operateur zur optischen Vergrösserung des Operationsgebietes. Die Operationstechnik ist hierbei so weit fortgeschritten, dass Vergrösserungen im Bereich von 50fach und darüber keine Seltenheit sind. Wesentlich während einer Operation ist, dass der Operateur alle wichtigen Informationen so schnell und eindeutig wie möglich übermittelt bekommt, um die Operation in einer möglichst kurzen Zeit abschliessen zu können. Da der Operateur vorzugsweise seine Augen möglichst wenig vom Okular des Operationsmikroskopes entfernt und man umgekehrt beim gesprochenen Wort Verständigungsschwierigkeiten erwarten kann, ist es naheliegend, wichtige Informationen wie z.B. Patientendaten, Mikroskopsteuerungs- oder Positionsangaben im Tubus visuell sichtbar zu machen.

Nach bekannten Techniken wird dies dadurch erreicht, dass die betreffenden Informationen auf einem Display dargestellt werden und das Bild dieses Displays über einen Strahlenteiler in den Tubus eingespiegelt wird. Weil der Anwender stets eine gute Lichtausbeute beim betrachteten Objekt wünscht, die vielfach durch hohe Beleuchtungsdichten am Objekt sichergestellt werden kann, ergibt sich beim Einspiegeln häufig das Problem der ausreichenden Lichtdichte des eingespiegelten bzw. überlagerten Bildes. Mit Röhrendisplays (CRT) findet sich dabei häufig kein Auslangen. Die Verwendung von LCD's mit starken Hintergrundbeleuchtungen bringt Nachteile im Bereich der Auflösung und auch bei dem Unterfangen, dünne Linien wiederzugeben, zumal die Pixelbreite der LCD's relativ gross ist und daher relativ breite Minimalliniendicken vorgegeben sind. Darüber hinaus bilden LCD-Pixel Raster, die Probleme bei der Randschärfe und der Auflösung erzeugen können.

Werden nun beispielsweise Randverbesserungen, Bildfärbungen, Kontrastverbesserungen oder andere möglichst dünne Markierungen gewünscht, die z.B. nach vorheriger Aufnahme mittels Videotechnik und mittels elektronischer Bildverarbeitung aufbereitet wurden, so kann es nachteiligerweise bei den bekannten Möglichkeiten zu unbefriedigenden Leistungen hinsichtlich der Helligkeit und/oder Liniendicke kommen. Eine Konturierung wäre wünschenswert, jedoch mit den Mitteln des Standes der Technik nicht optimal erzielbar.

Ein spezielles Gebiet für die Überlagerung von Bildern ergibt sich z.B. bei der Anwendung von Computertomographie (CT) oder Magnetresonanz bzw. Kernspintomographie (MRI) im Zusammenhang mit der Stereomikroskopie. Daten aus CT und MRI werden gewonnen, um vom Patienten ein Schnittbild des interessierenden Gebietes zu erhalten, das schlussendlich nach EDVmässiger Verarbeitung die Darstellung eines dreidimensionalen wirklichkeitsgetreuen Modelles auf einem Computermonitor (Stereobildschirm) ermöglicht. Durch solche dreidimensionale Bilder ist es den behandelnden Ärzten möglich, Art und Ausbreitung des kranken Gebietes besser zu lokalisieren. Häufig ist jedoch sowohl das aktuell gesehene Bild als auch die vorliegende dreidimensionale Darstellung von Röntgen- bzw. CT-Bilddaten nicht deutlich genug, um während der Operation das betreffende Gebiet genügend klar vom übrigen Gebiet abgegrenzt zu erkennen.

Damit diese Kennzeichnung optimal erfolgt, genügt eine Konturenüberarbeitung bzw. Konturendarstellung, die jedoch - wie schon erwähnt - möglichst hell und dünn sein sollte, um andere Details nicht zu überdecken.

Dies zu bewerkstelligen ist eine der Hauptaufgaben, die der Erfindung zugrunde liegen.

Gelöst wird diese Aufgabe beispielsweise durch die Anwendung des Verfahrens nach Anspruch bzw. einer Vorrichtung gemäss Anspruch 1.
Durch das Überlagern von einem ersten durch ein Hauptobjektiv (8) eines Mikroskopes gesehenen Bild durch wenigstens ein zweites Bild aus einem dünnen, gebündelten Lichtstrahl - insbesondere durch einen Laserstrahl, der in einer Ablenkvorrichtung so abgelenkt bzw. moduliert und über ein Einblendelement in den Strahlengang des Mikroskopes eingespiegelt wird, dass er das zweite Bild für ein Betrachterauge sichtbar darstellt - werden die beschriebenen Probleme beseitigt. Ein dünner Lichtstrahl - insbesondere ein Laserstrahl - kann nahezu beliebig dünn und hell erzeugt werden.

Die dafür neu erforderlichen Bauelemente gemäß Anspruch 1 sind relativ leicht in ein Mikroskop integrierbar. Einblendelemente und geeignete Lichtquellen - insbesondere Laser - sind dem Fachmann an sich bekannt. Sie wurden jedoch bisher offensichtlich trotz ihrer günstigen Eigenschaften für die angestrebten Effekte im Mikroskopiebereich nicht eingesetzt.

Im Sinne der Erfindung ist es dabei wesentlich, daß der dünne Lichtstrahl bzw. Laserstrahl durch die Optik direkt auf die Netzhaut geworfen wird

Die Schriften EP-A-0 562 742, EP-A-0 367 246 und EP-A-0 473 343 offenbaren Vorrichtungen, bei denen Bildinformationen mittels eines Laserstrahls direkt auf der Netzhaut abgebildet werden. Es ist jedoch zu bemerken, dass im Gegensatz zur Einspiegelung gemäss der US-A-4 722 056 bei diesen Vorrichtungen der Laserstrahl nicht innerhalb eines weiteren optischen abbildenden Systems geführt wird. Deshalb können diese Systeme der genannten Schriften nicht ohne weiteres für eine Abänderung bzw. Verbesserung der Lehre den erwähnten US-A in Betracht gezogen werden.

Im Rahmen der Erfindung liegen sowohl Varianten, bei denen der Lichtstrahl im Betriebszustand im Bereich einer Zwischenbildebene der Optik etwa parallel zur optischen Achse in Richtung des Okulars erstreckt ist, als auch Varianten, bei denen in der Zwischenbildebene eine Streuscheibe angeordnet ist, an der der Lichtstrahl streubar ist.

Unter Streuscheibe ist im Sinne der Erfindung dabei jedes optische Element zu verstehen, an dem ein dünner Lichtstrahl beim Auftreffen so gestreut wird, dass sein Berührungspunkt aus verschiedenen Blickwinkeln gesehen werden kann. Das könnte also auch eine nicht entspiegelte Glasplatte sein. Es kann aber auch z.B. ein Strahlenteiler sein, an dem an einer Fläche innerhalb der Optik ein streuender Belag aufgebracht ist, bzw. dessen eine Fläche aufgerauht ist.

Erfindungsgemäss können mittels Lichtstrahl und Ablenkvorrichtung beliebige bildhafte Informationen dem Betrachter zugespielt werden. Gemäss einer speziellen Ausbildung der Erfindung ist die Ablenkvorrichtung und/oder die Lichtquelle durch eine Bildverarbeitungsvorrichtung zur Konturendarstellung bzw. Konturennachzeichnung gesteuert, die mit einer Bildaufnahmevorrichtung gekoppelt ist, welche über ein weiteres Einblendelement mit der Optik gekoppelt ist. Derart ist eine direkte Rückkopplung zwischen gesehenem Bild des Objektes und mittels Strahl dargestelltem Bild möglich. Bedingt durch die Helligkeit eines gebündelten Lichtstrahles ist diese Variante eine vorteilhafte. Trotz hellem Operationsgesichtsfeld sieht der Operateur deutlich unterstrichen den hervorzuhebenden Bereich. Dies natürlich auch bei den Varianten, bei denen nicht nur das optisch gesehene Bild die Grundlage für die Bilddarstellung des Strahles ist, sondern auch die objektpositionsgleich wirkenden Diagnosedaten aus einer Diagnosedateneinrichtung (z.B. einer CT-, MRI-, PE-, Ultraschall-Einrichtung o.dgl.).

Bei letzteren Varianten ist bevorzugt je wenigstens ein Strahlengang pro Betrachterauge vorgesehen (Stereomikroskop), wobei in jedem Strahlengang je eine linke bzw. rechte - zur jeweils anderen - perspektivisch versetzte Teilbildinformation aus einer oder je einer Ablenkvorrichtung über je ein Einblendelement einspiegelbar ist, wobei die Ablenkvorrichtung(en) durch eine Bilderkennungs- und/oder Bildverarbeitungsvorrichtung gesteuert ist bzw. sind.

Unter einer Bilderkennungsvorrichtung ist ein System zu verstehen, das die Identität der durch verschiedene Betrachtungsgeräte betrachteten Objekten zu erkennen vermag und derart ein positionsrichtiges Überlagern von Bilddaten ermöglicht. Hierzu wird ausdrücklich auf die Schweizer Patentanmeldung CH3932/94-0 vom 23.12.1994 der Anmelderin verwiesen, in der eine besonders geeignete Vorrichtung beschrieben ist, bei der Bilddaten nicht nur positionsrichtig, sondern auch optisch korrigiert und aneinander angepasst überlagert werden können. Eine Kombination der beiden Erfindungen ist vorteilhaft.

Das bevorzugte Verfahren in diesem Zusammenhang ergibt sich, wenn die Ablenkvorrichtung durch aus dem durch das Mikroskop betrachteten Objekt gewonnene Bildinformationen gesteuert wird, so dass beispielsweise Konturen von Objektdetails nachgezeichnet oder Objektdetails mit Gitternetzlinien o.dgl. am tatsächlichen Ort des Objektdetails im Sehfeldbereich dargestellt werden. Solche Gitternetzlinien wirken natürlich vor allem bei Stereomikroskopen ideal, wenn durch sie räumlich bzw. plastisch ein spezielles Objektdetail - z.B. ein Tumor - nachgezeichnet wird.

Gemäss einer Weiterbildung der Erfindung ist der Lichtquelle ein stufenlos regelbares Lichtventil vorgeschaltet, so dass ein Anwender die Helligkeit des mittels Strahls eingeblendeten Bildes regulieren kann. Bei der Anwendung von herkömmlichen Lampen können natürlich auch diese über die Stromzufuhr helligkeitsreguliert werden. Insbesondere bei Lasern empfiehlt sich jedoch die erwähnte Variante.

Der Bedienkomfort ist gesteigert, wenn auch die Lichtfarbe der Lichtquelle bzw. des Lasers einstellbar ist, was mit dem Lichtquellen- bzw. Laser-Fachmann bekannten Massnahmen möglich ist.

Weitere Vorteile ergeben sich nach weiteren besonderen Ausgestaltungen der Erfindung, bei denen das Betrachtete (Hintergrund-)Bild eine Rückwirkung auf das überlagerte Bild ausübt. Hier geht man erfindungsgemäss nach zwei Gesichtspunkten vor: Relative Helligkeit einzelner Bildpunkte zueinander und Gesamthelligkeit des Bildes begrenzt durch mögliches Adaptionsverhalten.

In der Schweizer Patentanmeldung CH1091/94-3 vom 11.04.1994 ist eine Anordnung beschrieben, die es ermöglicht, oben erwähnte Überlagerungen und Datenadaptierungen möglichst schnell bzw. in Echtzeit zu ermöglichen. Eine Kombination der vorliegenden Lehre mit der Lehre der erwähnten Anmeldung bringt daher weitere Vorteile. Insofern gilt der Inhalt der erwähnten Patentanmeldung als im Rahmen dieser Offenbarung liegend.

Weitere Details und Ausführungen der Erfindung sowie Varianten dazu ergeben sich aus der Zeichnung. Die dort dargestellten Figuren zeigen beispielhaft:
- Fig.1: einen Prinzipaufbau eines erfindungsgemässen Mikroskops ohne Streuscheibe;
- Fig.2: eine Variante zu Fig.1 mit Streuscheibe und Patientendateneinspiegelung;
- Fig.3: eine Variante mit Ein- und Ausspiegelung von zusätzlichen Bilddaten und einer Rückkopplung dazwischen;
- Fig.4: ein Stereomikroskop mit 3-D-Bildüberlagerung und
- Fig.5: einen Aufbau mit einem kleinen beweglichen Spiegel im Strahlengang.

Die Figuren werden zusammenhängend und übergreifend beschrieben. Gleiche Bezugszeichen bedeuten gleiche Bauteile. Gleiche Bezugszeichen mit unterschiedlichen Indizes bedeuten ähnliche bzw. funktionsähnliche Bauteile. Die Erfindung ist auf die dargestellten Ausführungsbeispiele nicht eingeschränkt. Vor allem in Kombination mit den Lehren der Schweizer Patentanmeldungen CH949/94-2 (u/Z: R-P-3112-CH), CH1525/94-0 (u/Z: R-P-3138-CH), CH1295/94-8 (u/Z: R-P-3139-CH), CH1088/94-3 (u/Z: R-P-3140-CH), CH1089/94-5 (u/Z: R-P-3142-CH), CH1090/94-1 (u/Z: R-P-3165-CH), CH1091/94-3 (u/Z: R-P-3174-CH), CH1092/94-5 (u/Z: R-P-3175-CH), vor allem auch CH3932/94-0 (u/Z: R-P-3141-CH),lassen sich noch beliebige Varianten darstellen. Sie alle fallen für den Zweck einer Kombination ihrer Lehren unter den Offenbarungsinhalt dieser Anmeldung. Die angefügte Bezugszeichenliste ist dementsprechend fortlaufend geführt.

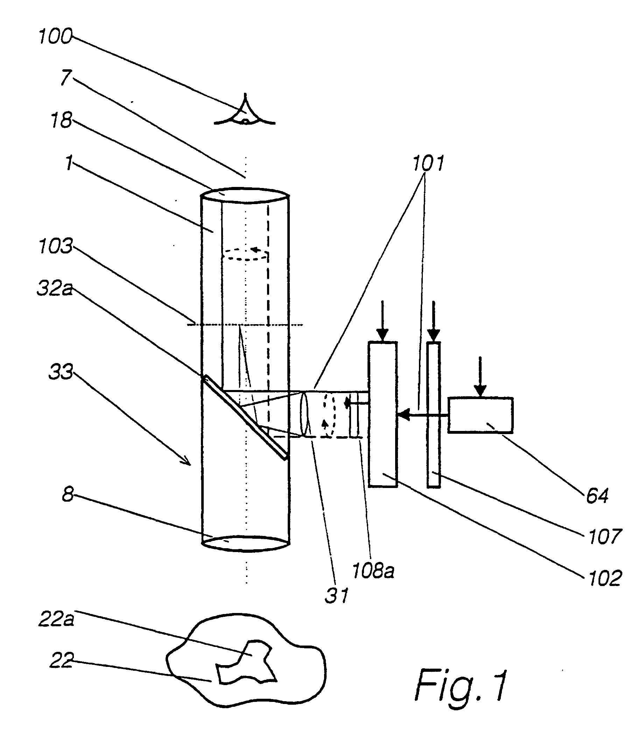

Eines der Prinzipien der Erfindung wird in Fig.1 verdeutlicht:

Ein Strahlengang 1 mit einer Vergrösserungsoptik 33, von der symbolisch nur zwei Linsen , nämlich ein Hauptobjektiv 8 und ein Okular 18, dargestellt sind, verfügt über einen schräggestellten Strahlenteiler 32a, durch den sowohl die Bildinformation durch das Hauptobjektiv 8 als auch eine seitlich eingespiegelte Bildinformation zum Okular 18 gelenkt wird. Der Strahlenteiler kann ein teildurchlässiger Spiegel o.dgl. sein. Er kann eventuell auch miniaturisiert als kleiner Spiegel ausgebildet sein, der auf einer zur optischen Achse 7 senkrechten Glasplatte aufgeklebt ist, wie dies in der Schweizer Patentanmeldung CH1092/94-5 vom 11.04.1994 dargestellt ist, wobei ein solch letzterer Aufbau stets mit einer Divergenz der Strahlen 101 funktioniert, die gegebenenfalls zusätzlichen rechnerischen Aufwand bei der Strahlablenkung erfordert. Die seitlich eingespiegelte Bildinformation besteht erfindungsgemäss aus einem Bild eines dünnen, gebündelten Lichtstrahls 101 - insbesondere eines Laserstrahls - , der mit der erforderlichen Geschwindigkeit wiederholt die bildnerisch darzustellenden Linien abfährt, so dass für einen Betrachter ein zusammenhängendes Bild - z.B. Linien, Zahlen, Buchstaben, Symbole, Flächen usw. - sichtbar werden. Diese werden auf einer Streuscheibe 108a gestreut und zeichnen sich dort durch eine gute - in Abhängigkeit von der Laserleistung nahezu unbegrenzte - Helligkeit und somit deutlichen Kontrast gegenüber dem durch das Hauptobjektiv 8 gesehenen Bild aus. Das Bild des gestreuten Laserstrahles wird mittels Linse 31 über den Teilerspiegel 32a in eine Zwischenbildebene 103 des Tubus abgebildet. Die Ablenkung bzw. Bewegung des Strahls ergibt sich durch eine an sich bekannte Ablenkvorrichtung 102, die über gesteuert bewegbare Spiegel o.dgl. verfügt und derart einen in sie eingestrahlten Strahl 101 ablenken kann. Es versteht sich von selbst, dass gleichzeitig auch mehrere Strahlen mit gegebenenfalls auch mehreren Ablenkvorrichtungen vorgesehen sein könnten. Auch ist es denkbar, bei Bedarf mehrere Laserstrahlen auf der Streufläche zusammenlaufen zu lassen, um die Energiedichte und damit Helligkeit zu erhöhen. Die Bewegung des Strahls 101 ist symbolisch durch einen strichlierten Drehpfeil dargestellt. Gewonnen wird der Strahl 101 aus einer Lichtquelle 64, insbesondere aus einem Laser, der natürlich auch in die Ablenkvorrichtung integriert sein könnte.

Bei dem gezeigten Ausführungsbeispiel ist auch ein Lichtventil 107 dargestellt, das die Helligkeit des Strahls 101 regulierbar macht. Ablenkvorrichtung 102, Lichtventil 107 und Lichtquelle 64 sind vorzugsweise von aussen willkürlich steuerbar, um das durch den Strahl 101 darzustellende Bild hinsichtlich der Strahlqualität für den Betrachter, dessen Auge 100 dargestellt ist, optimal zu gestalten.

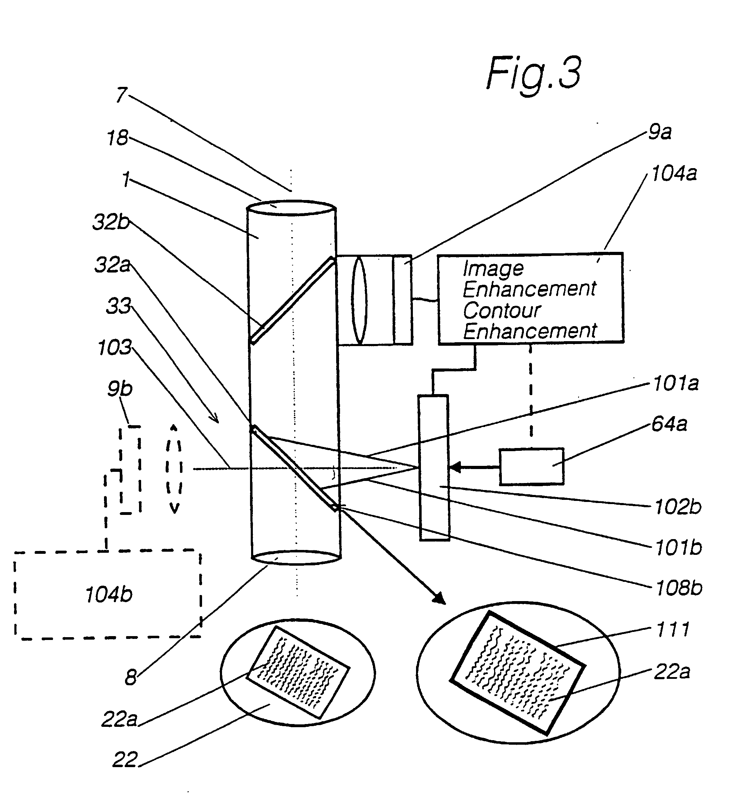

Bei den Ausführungsbeispielen gemäss Fig.1 und Fig.3 wird der Strahl 101 so abgelenkt, dass er das durch ihn entstehende Bild über das Okular 18 unmittelbar in das Betrachterauge 100 bzw. auf dessen Netzhaut abbildet. Die verwendete Lichtquelle 64 kann demzufolge relativ lichtschwach sein. Im Bereich einer Zwischenbildebene 103 der Optik 33 verlaufen die Strahlen 101 somit vorzugsweise parallel zur optischen Achse 7 der Optik 33. Es ist aber nicht wesentlich, dass der Strahl 101 an der gezeigten Stelle eingeblendet wird. Denkbar ist auch eine Variante, bei der der Strahl erst nach dem Okular 18 in das Betrachterauge 100 gelenkt wird. Insbesondere in Kombination mit der Lehre der Schweizer Patentanmeldung CH1092/94-5 ist diese Variante sinnvoll, da durch sie der nötige Augenabstand zum Okular nicht unnötig vergrössert wird.

Das betrachtete Objekt ist symbolisch mit 22 dargestellt, wobei der Sinn dieses Aufbaus z.B. darin liegt, ein Objektdetail 22a mit einer kontraststarken, hellen Linie zu umzeichnen. Im Rahmen der Erfindung liegt daher auch jene Variante, bei der der Strahl 101 nicht direkt ins Betrachterauge 100, sondern direkt auf das Objekt 22 abgebildet wird, wobei der Strahlenteiler 32a dann natürlich umgekehrt wirken muss.

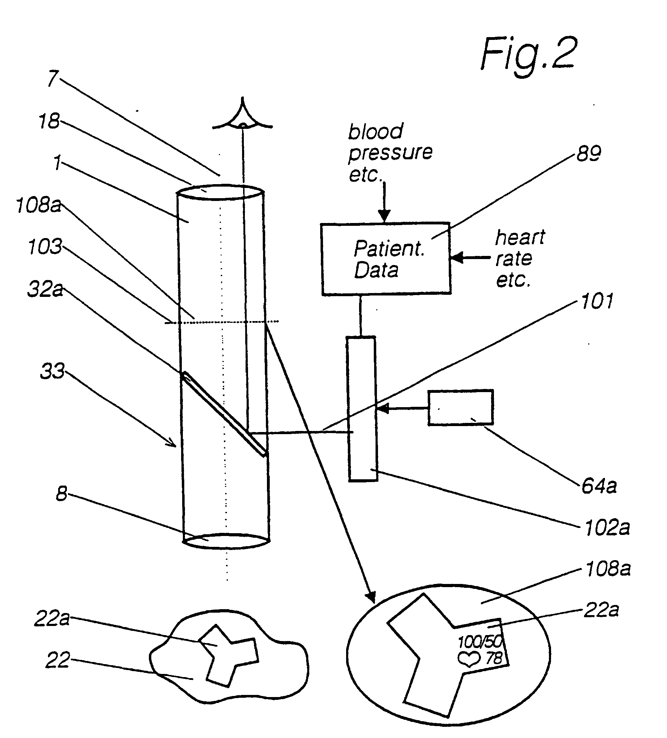

Mit dem Strahl können beispielsweise Patienteninformationsdaten wie Blutdruck, Herzfrequenz usw. in anschaulicher Form dargestellt werden, wie in Fig.2 angedeutet ist.

Unterschiedlich zur ersten Variante ist dort ein Laserstrahl 101 direkt in das Auge gerichtet, wie symbolisch angedeutet ist. Der Optiker kennt die erforderlichen Massnahmen, um den Strahl 101 direkt an der Netzhaut richtig abzubilden. Eine Streuscheibe kann derart entfallen und der Strahl kann mit entsprechend weniger Energie eine grosse Helligkeit und Kontraststärke aufbringen. Das durch die Ablenkvorrichtung 102a vorgegebene Bild entsteht somit direkt an der Netzhaut und wird eben dort dem durch das Hauptobjektiv 8 gesehenen Bild überlagert. Bei diesem Aufbau ist es unerheblich, welchen Winkel die Strahlen zu der optischen Achse 7 haben, sofern sie nur am gewünschten Ort auf der Netzhaut auftreffen. Altemativ zu diesem Aufbau könnte in der Zwischenbildebene 103 ein Streugitter eingesetzt sein, an dem lediglich der Wellenlängenbereich des Laserlichtes gestreut, die anderen Lichtwellenlängenbereiche jedoch ungehindert passieren, so dass es trotz Streuscheibe zu keiner nennenswerten Abdunkelung des gesehenen Bildes unter dem Hauptobjektiv 8 kommt.
Im dargestellten Beispiel sind im Bereich des gesehenen Objektdetails 22a der Blutdruck und die Herzfrequenz dargestellt. Diese Patienteninformationen werden von bekannten Messgeräten gewonnen und gegebenenfalls über eine Datenaufbereitungseinheit 89 - vgl. die Schweizer Patentanmeldung CH1091/94-3 - so aufbereitet, dass geeignete Steuerdaten der Ablenkvorrichtung 102a zugeführt werden können, um einen raschen Istzeit-Betrieb zu ermöglichen.

Bei dem in Fig.3 dargestellten Beispiel geht es um eine bildverarbeitende (Video-)Auswertung eines durch das Hauptobjektiv gesehenen Bildes, z.B. Objektdetail 22a durch eine Bildverarbeitungsvorrichtung 104a, die mit einer Bildaufnahmevorrichtung 9a (z.B. CCD) gekoppelt ist. Die Bildaufnahmevorrichtung 9a ist über eine Abbildungsoptik und über einen Strahlenteiler 32b mit dem Strahlengang 1 gekoppelt, so dass die Bildverarbeitungsvorrichtung 104a das betrachtete Objektdetail 22a erkennt. Strichliert sind Bildaufnahmevorrichtung 9b und Bildverarbeitungsvorrichtung 104b dargestellt, die zusätzlich oder alternativ zu 9a und 104a vorgesehen sein können. In der ausgezogenen Variante (9a,104a) steht der Bildverarbeitungsvorrichtung 104a neben dem Bild des Objektdetails 22a auch das Bild aus der Ablenkvorrichtung 102b zur Verfügung, das umgekehrt von der Vorrichtung 104a entworfen wird. Eine Nachkorrektur ist somit leicht möglich. Im vorliegenden Beispiel geht es darum, Konturen am Objektdetail 22a zu erkennen und diese mittels Strahlüberlagerung (101) zu verstärken. Dies erleichtert es einem Operateur, z.B. betroffene Gebiete schneller und deutlicher auszumachen.

Der Strahlenteiler 32a ist in diesem Beispiel etwa mittig um eine Zwischenbildebene 103 angeordnet und selbst mit einer geringfügig streuenden Oberfläche 108b versehen, so dass eine zusätzliche Streuscheibe entfällt.

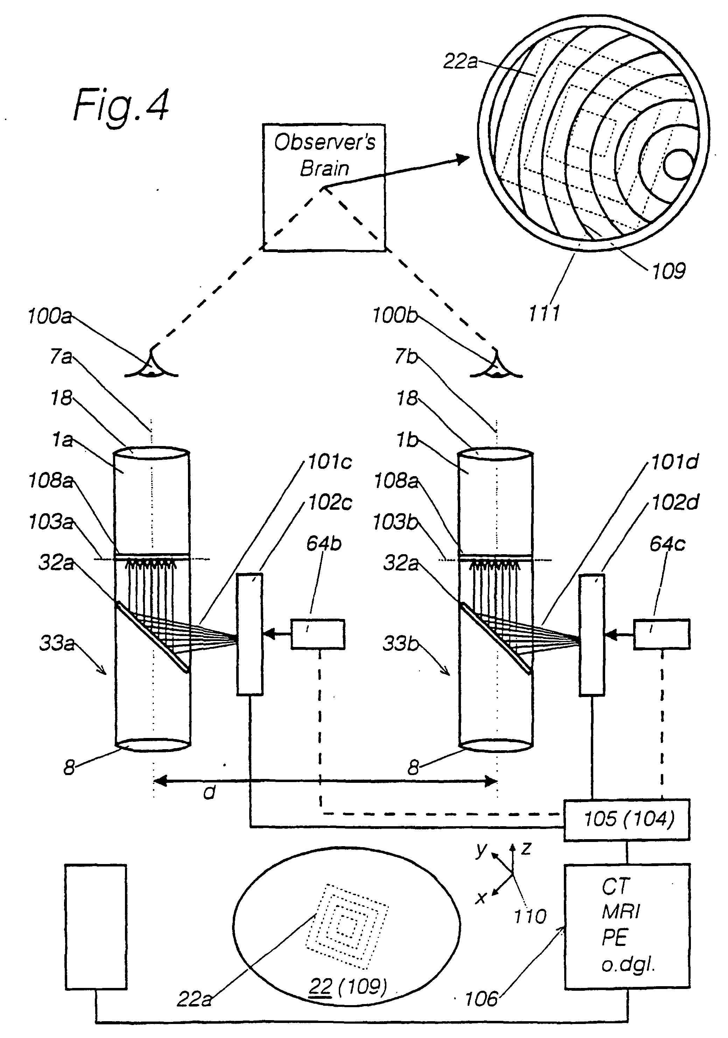

Bei dem beispielhaften Stereomikroskop gemäss Fig.4 sind wieder Streuscheiben 108a vorgesehen, auf denen pro Strahlengang 1a,1b zur jeweiligen optischen Achse 7a,b um die Parallaxe versetzte Teilbilder dargestellt sind, die im Gehirn des Betrachters zu einem 3-D-Bild zusammengesetzt werden. Die Ablenkvorrichtungen 102c,d werden dazu von einer Bilderkennungsvorrichtung 105 eventuell mit einer Bildverarbeitungsvorrichtung 104 angesteuert, die ihre Bildinformationen - eventuell über eine nicht dargestellte Datenaufbereitungseinheit (89) und/oder über einen Bildspeicher - aus einer 3-D-Bilddatenaufzeichnungseinheit bzw. Diagnosedateneinrichtung 106 erhält. Letztere operieren bevorzugt nicht im sichtbaren Wellenlängenbereich wie die Mikroskopstrahlengänge, sondern mit Röntgenstrahlen, Magnetwechselfeldern, Positronenstrahlen, Ultraschall o.dgl.

Mit diesem Aufbau lassen sich somit z.B. aus den erwähnten Patientendaten berechnete dreidimensionale Gitter(netz)linien einem dreidimensional gesehenen oder theoretisch zu sehenden Objektdetail 22a überlagern, so dass ein Operateur wieder deutlich und hell den ihn interessierenden Bereich - mit dünnen Linien unterstrichen - zu sehen bekommt.

Selbstverständlich sind auch beliebige Kombinationen von überlagerten Bildern im Rahmen der Erfindung enthalten, wie alphanumerische Angaben, Konturenverstärkung und Gebietseinkreisung.

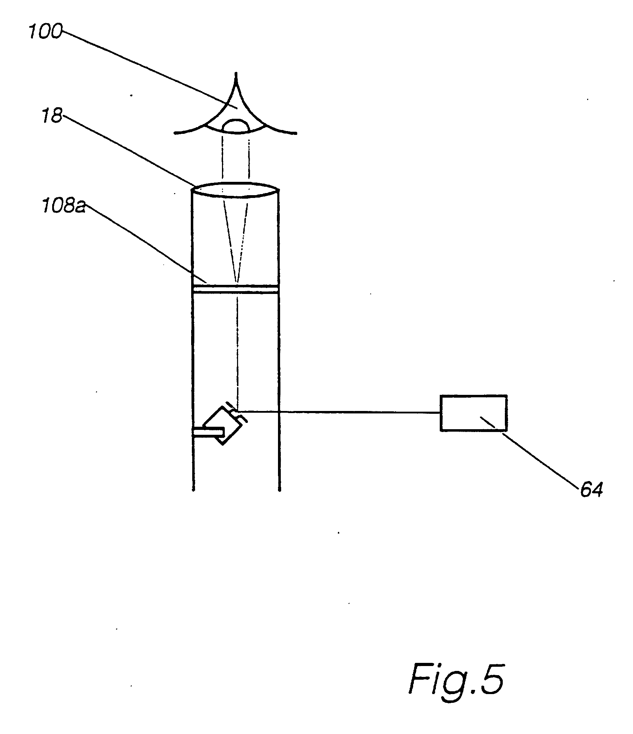

Die Variante gemäss Fig.5 arbeitet mit einer Mikrospiegelumlenkeinheit mit Antrieb im Tubus 33, die einen Laserstrahl 101 positionsrichtig gegen das Betrachterauge 100 lenkt.

Nicht näher dargestellt, aber dem Fachmann geläufig, sind Varianten mit farbigen Lasern oder mit Elektronenstrahlen, die geeignete Fluoreszenzscheiben o.dgl. zum figuralen Aufleuchten bringen. Solche Aufbauten umfassen gegebenenfalls auch Vektorenbildschirme, wo Linien durch den Strahl jeweils öfter nachgefahren werden.

Weiterhin bekannt sind auch Varianten, bei denen die Lichtstrahlen anstelle unmittelbar dem Auge zuerst dem betrachteten Objekt zugeführt und an diesem zur Streuung gebracht werden.

### Bezugszeichenliste

Diese Bezugszeichenliste enthält auch Bezugszeichen von Figuren, die in den oben erwähnten Anmeldungen beinhaltet sind, da diese - wie erwähnt - als im Rahmen dieser Erfindung liegend zu Kombinationszwecken mitgeoffenbart gelten. Insbesondere betrifft dies die Mikroskope mit speziellen Strahlengängen und Strahlenteilern und die Vorrichtungen zum Messen der Vergrösserung und des Abstandes vom Mikroskop zum Objekt sowie Mikroskope für stereotaktische Operationen usw.
- 1: a,b erster Strahlengang
- 2: a,b zweiter Strahlengang (geometrisch übereinander gelegte erste Strahlengänge)
- 3: mechano-optisches Schaltelement
3a,3b,3c undurchlässige und vorzugsweise verspiegelte Blende
3d LCD-Shutter-Element
3e mikromechanische Lamellenspiegelkonstruktion
3f LCD Wechselshutterelement
- 4: Strahlenteiler
4a,4b Strahlenteiler
4c Strahlenteiler für Messstrahlausblendung
- 5: Scheibe
5a halbkreisförmige Fläche
5b Restfläche der Scheibe 5
5c Kreissegmentflächen 5d
- 6: Achse für Scheibe
- 7: Mittelachse
7a,7b Mittelachse
- 8: xHauptobjektiv
- 9a: elektronische Bildaufnahmevorrichtung
- 10: Display
10a Display
- 11: a,b Spiegel
- 12: a,b,c Verstelleinrichtung
- 13: Zoom
- 14: a,b Motor
- 15: Reziprokantrieb
- 16: Zuleitung
- 17: Lichtquelle
- 18: Okular
- 19: Umlenkspiegel
- 20: Schubstange
- 21: starrer Spiegel
- 22: Objekt
22a Objektdetail
- 23: a,b, a',b',c,d Planplatte
- 24: Schwenkantrieb
- 25: x Gestänge
- 26 27 28 29 30: Lamellenspiegel von 3e
- 31: Tubuslinse
- 32: Einblendelement
32a Strahlenteiler
32b Spiegel
32c zweites Einblendelement
- 33: Vergrösserungsoptik
- 34: Pfeile
- 35: weiterer Spiegel
- 36: Stellantrieb
- 37: Balken
- 38: a,b Umlenkspiegel
- 39: Retroprisma
- 40: Ausgleichsgewicht
- 41: Trägerplatte a,b,c: prismatische mit integriertem Spiegel
- 42: Farbfilter
- 43: Intervallschalter
- 44: Mikroprozessor
- 45: Messarray a
- 46: Referenzarray a
- 47: Modul für Bilddatenübertragung
- 48: Fremdbilddaten-Input
- 49: Stellmotor für Zoom 13; a,b
- 50: Verbindungsleitungen a-g
- 51: Vergrösserungsanzeige a,b,c
- 52: Kurvenscheibe
- 53: Kopplung
53a zwischen Stellmotor 49 und Zoom 13
53b zwischen Kurvenscheibe 52 und Vergrösserungsanzeige 51b
- 54: mechanischer Abgriff
- 55: a,b Zeiger
- 56: Laser
- 57: Messstrahl a,b,c,c1
- 58: Referenzstrahl
- 59: Pfeile für Verschiebbarkeit des Einblendelementes 32
- 60: Mikroskopstrahlengang a-e
- 61: erstes Umlenkelement a
- 62: Fokussierelement a,b
- 63: Lichtleiterendstück a,b
- 64: Lichtquelle a
- 65: zweites Umlenkelement
- 66: Sensor
- 67: Distanzbereich a
- 68: Verbindungsleitung
- 69: Distanzmesssystem
- 70: Verbindung
- 71: Vergrösserungsmesseinheit
- 72: Positionsbestimmungssystem a,b
- 73: Interferometer
- 74: halbdurchlässiger Spiegel
- 75: Reflektor
76 Detektor
77 elektromechanisches Verstellelement
78 Interferometersteuerung
79 Gitter
80 Detektor-CCD
81 Stufen
82 Mikroskop
83 Anordnung zur Vergrösserungsmessung des Mikroskopes
84 Anordnung zur Entfernungsmessung Objekt/Mikroskop
85 Positionsmesssystem zur Bestimmung der Absolutlage des Mikroskopes im Raum, um daraus nach Kenntnis der Entfernung Objekt/Mikroskop auch auf die Lage des Sehfeldes am Objekt schliessen zu können
86 Toolbox für verschiedene Anwenderprogramme
87 Befehlssteuerorgan (Computermouse)
88 Befehlssteuerorgan zur Bewegungssteuerung des Mikroskopes (z.B. Fussschalter)
89 Datenaufbereitungseinheit
90 Computer (Workstation)
91 Steuerschalter für Mikroskop
92 elektromechanische Steuereinheit für Mikroskop (Zoom, Fokus etc.)
93 Ojekt
94 zweite Vorrichtung (z.B. MRI- oder CT-Gerät)
95 Überlagerungseinrichtung
96 Gelenk am Stativ
97 adaptive Regelvorrichtung
98 a,b Erkennungseinheit
99 Speicher
100 a,b Betrachterauge
101 a,b,c Strahlen
102 a,b,c,d Ablenkvorrichtung
103 a,b Zwischenbildebene
104 Bildverarbeitungsvorrichtung
105 Bilderkennungsvorrichtung
106 Diagnoseeinrichtung
107 Lichtventil
108 a,b Streuscheibe
109 Testobjekt
110 Koordinatensystem
111 verstärkte Konturenlinie
112 Mikrospiegelumlenkeinheit
b Abstand der Messstrahlen 57a und 57b
b' Abstand der Messstrahlen 57a und 57b am Messarray
d 1,2 Stereobasis

## Patentansprüche

1. Mikroskop mit wenigstens einem Strahlengang (1) und einer Optik (33) entlang einer optischen Achse (7) und mit einem Einblendelement (32a) für das Einspiegeln von Bildinformationen für ein Betrachterauge (100), **dadurch gekennzeichnet, dass** dem Einblendelement (32a) eine Lichtquelle (64) - insbesondere ein Laser - zur Erzeugung eines dünnen, gebündelten Lichtstrahles (101) zugeordnet ist, dessen Lichtenergie in modulierter Form mittelbar oder unmittelbar durch eine Ablenkvorrichtung (102) über das Einblendelement (32a) in Einblendrichtung der Bildinformation in den Strahlengang (1) einblendbar ist, wobei die Ablenkvorrichtung (102) so angepaßt ist, daß der Lichtstrahl (101) im Anwendungszustand die Bildinformation direkt an der Netzhaut des Anwenderauges erzeugt und dort dem durch das Hauptobjektiv gesehenen Bild überlagert.

2. Mikroskop nach einem der vorhergehenden Ansprüche, **dadurch gekennzeichnet, dass** der Ablenkvorrichtung (102) und/oder der Lichtquelle (64) eine diese steuernde Bildverarbeitungsvorrichtung (104) zur Konturendarstellung zugeordnet ist, die mit einer Bildaufnahmevorrichtung (9) gekoppelt ist, welche über ein weiteres Einblendelement (32b) mit der Optik (33) gekoppelt ist.

3. Mikroskop nach einem der vorhergehenden Ansprüche, mit je wenigstens einem Strahlengang (1a,b) pro Betrachterauge (100a,b)- im folgenden Stereomikroskop genannt-, **dadurch gekennzeichnet, dass** in jedem Strahlengang (1a,b) je eine
- linke bzw. rechte zur jeweils anderen - perspektivisch versetzte Teilbildinformation aus einer oder je einer Ablenkvorrichtung (102) über je ein Einblendelement (32a) einspiegelbar ist, wobei die Ablenkvorrichtung(en) (32) durch eine mit ihr verbundene Bilderkennungs- und/oder Bildverarbeitungsvorrichtung (105,104) gesteuert ist bzw. sind, die von einer getrennten, jedoch objektpositionsgleich wirkenden Diagnosedateneinrichtung (106) ansteuerbar ist.

4. Mikroskop nach einem der vorhergehenden Ansprüche, **dadurch gekennzeichnet, dass** der Lichtquelle (64) ein stufenlos regelbares Lichtventil (107) vorgeschaltet ist.

5. Mikroskop nach einem der vorhergehenden Ansprüche, **dadurch gekennzeichnet, dass** die Lichtfarbe der Lichtquelle (64) bzw. des Lasers einstellbar ist, bzw. dass mehr als eine Laserlichtquelle mit unterschiedlichen Lichtfarben vorgesehen sind, denen je eine Ablenkeinheit zugeordnet ist.

6. Mikroskop nach einem der vorhergehenden Ansprüche, **dadurch gekennzeichnet, dass** als Umlenkelement wenigstens eine Mikrospiegelanordnung vorgesehen ist, die direkt im Tubus angeordnet ist.

7. Operationsmikroskop mit einem Mikroskop nach einem der vorhergehenden Ansprüche.

8. Verfahren für das Überlagern von einem ersten durch ein Hauptobjektiv (8) eines Mikroskopes (82) gesehenen Bild durch wenigstens ein zweites Bild, **dadurch gekennzeichnet, dass** ein dünner, gebündelter Lichtstrahl (101) aus einer Lichtquelle (64) in einer Ablenkvorrichtung (102) so abgelenkt bzw. moduliert und über ein Einblendelement (32a) in den Strahlengang (1) des Mikroskopes (82) eingespiegelt wird, dass er das zweite Bild für ein Betrachterauge (100) sichtbar darstellt, indem der Lichtstrahl (101) direkt an der Netzhaut des Betrachterauges (100) das zweite Bild erzeugt.

9. Verfahren nach Anspruch 8, **dadurch gekennzeichnet, dass** die Ablenkvorrichtung (102) durch aus dem durch das Mikroskop (82) betrachteten Objekt (22) gewonnene Bildinformationen gesteuert wird, so dass beispielsweise Konturen von Objektdetails (22a) nachgezeichnet oder Objektdetails (22a) mit Gitternetzlinien o.dgl. am tatsächlichen Ort im Sehfeldbereich dargestellt werden.

10. Verfahren nach Anspruch 9, **dadurch gekennzeichnet, dass** die Bildinformationen vorzugsweise dreidimensional aus einer vom Mikroskop unabhängigen Diagnosedateneinrichtung (106) - z.B. einer Röntgen-, CT-, MRI-, PE-, Ultraschall- Einrichtung - gewonnen werden, wobei sowohl das Mikroskop (82) als auch die Diagnosedateneinrichtung (106) - vorzugsweise mittels Testmessung an einem genormten Testobjekt (109) - einem gemeinsamen Koordinatensystem (110) zugeordnet wird.

11. Verfahren nach Anspruch 10, **dadurch gekennzeichnet, dass** die Bilddaten mittels einer Korrektureinheit aufeinander abgestimmt sind, so dass eine vollständige Übereinstimmung in der Objektdetailposition erzielt wird.

## Claims

1. Microscope with at least one beam path (1) and an optical system (33) along an optical axis (7) and with a mixing-in element (32a) for the reflection in of image data for an eye (100) of an observer, **characterised in that** the mixing-in element (32a) is associated with a light source (64) - particularly a laser - for generation of a thin, focussed light beam (101), the light energy of which can be mixed into the beam path (1) in modulated form directly or indirectly by a deflecting device (101) via the mixing-in element (32a) in the mixing-in direction of the image information, wherein the deflecting device (102) is so adapted that the light beam (101) in the use state generates the image information directly at the retina of the eye of the user and superimposes there the image seen through the main objective.

2. Microscope according to one of the preceding claims, **characterised in that** the deflecting device (102) and/or the light source (64) is or are associated with an image processing device (104), which controls this or these, for representation of contours, the image processing device being coupled with an image recording device (9) which is coupled with the optical system (33) by way of a further mixing-in element (32b).

3. Microscope according to one of the preceding claims, with at least one beam path (1a, b) for each eye (100a, b) of the observer, the microscope being termed stereo microscope in the following, **characterised in that** a respective item of partial image information from one or each deflecting device (102) can be reflected by way of a respective mixing-in element (32a) into each beam path (1a, b) to be perspectively offset to the left or the right relative to the respective other item, wherein the deflecting device or devices (32) is or are controlled by an image recognition and/or image processing device (105, 104), which is or are connected therewith and controllable by a diagnostic data device (106) which is separate, but which acts with the same object position.

4. Microscope according to one of the preceding claims, **characterised in that** a steplessly regulable optical valve (107) is arranged in front of the light source (64).

5. Microscope according to one of the preceding claims, **characterised in that** the light colour of the light source (64) or the laser is settable or that more than one laser light source with different light colours are provided, with each of which a respective deflecting unit is associated.

6. Microscope according to one of the preceding claims, **characterised in that** at least one micro-reflecting arrangement, which is arranged directly in the barrel, is provided as deflecting element.

7. Surgical operation microscope with a microscope according to one of the preceding claims.

8. Method for superimposition on a first image, which is seen through a main objective (8) of a microscope (82), of at least one second image, **characterised in that** a thin, focussed light beam (101) from a light source (64) is so deflected or modulated in a deflecting device (102) and reflected into the beam path (1) of the microscope (82) by way of a mixing-in element (32a) that it visibly represents the second image for an eye (100) of an observer **in that** the light beam (101) generates the second image directly at the retina of the eye (100) of the observer.

9. Method according to claim 8, **characterised in that** the deflecting device (102) is controlled by image data obtained from the object (22) observed through the microscope (82), so that, for example, contours of object details (22a) are drawn in subsequently or object details (22a) are illustrated by grid lines or the like at the actual location in the field-of-view region.

10. Method according to claim 9, **characterised in that** the image data are obtained preferably three-dimensionally from a diagnostic data device (106), for example an X-ray, CT, MRI, PE or ultrasound device, independent of the microscope, wherein a common coordinate system (101) is associated with both the microscope (82) and the diagnostic data device (106), preferably by means of test measurement at a standardised test object (109).

11. Method according to claim 10, **characterised in that** the image data are matched to one another by means of a correction unit so that a complete agreement in object detail position is achieved.

## Revendications

1. Microscope avec au moins une marche des rayons (1) et une optique (33) le long d'un axe optique (7) et avec un élément d'ouverture en fondu (32a) pour l'introduction par réflexion d'informations d'image pour l'oeil d'un observateur (100), **caractérisé en ce qu'**il est associé à l'élément d'ouverture en fondu (32a) une source de lumière (64) - notamment un laser - pour produire un rayon de lumière mince en faisceau (101) dont l'énergie lumineuse peut être introduite sous une forme modulée directement ou indirectement par un dispositif de déflexion (102) par l'élément d'ouverture en fondu (32a) dans la direction d'ouverture de l'information d'image dans la marche des rayons (1), où le dispositif de déflexion (102) est adapté de façon que le rayon de lumière (101) produit à l'état d'application l'information d'image directement à la rétine de l'oeil de l'utilisateur et y superpose à l'image vue à travers l'objectif principal.

2. Microscope selon l'une des revendications précédentes, **caractérisé en ce qu'**il est associé au dispositif de déflexion (102) et/ou à la source de lumière (64) un dispositif de traitement d'images (104) commandant ceux-ci pour la représentation du contour, qui est couplé avec un dispositif de prise d'images (9) qui est couplé par un élément d'ouverture en fondu additionnel (32b) à l'optique (33).

3. Microscope selon l'une des revendications précédentes, avec respectivement au moins une marche des rayons (1a, b) par oeil d'observateur (100a, b) - désignée ci-après par stéréo-microscope -, **caractérisé en ce que** peut être introduit par réflexion dans chaque marche des rayons (1a, b) respectivement une information d'image partielle - gauche ou droite décalée respectivement en perspective vers l'autre à partir d'un ou respectivement un dispositif de déflexion (102) par respectivement un élément d'ouverture en fondu (32a), où le ou les dispositifs de déflexion (32) sont commandés par un dispositif de reconnaissance et/ou de traitement d'images (105, 104) relié à ceux-ci qui peut être commandé par une installation de diagnostic de données (106) séparée, cependant à effet de position d'objet identique.

4. Microscope selon l'une des revendications précédentes, **caractérisé en ce qu'**il est disposé en amont de la source de lumière (64) une vanne de lumière (107) réglable progressivement.

5. Microscope selon l'une des revendications précédentes, **caractérisé en ce que** la couleur de lumière de la source de lumière (64) respectivement du laser est réglable, respectivement **en ce que** sont prévues plus d'une source de lumière laser de couleurs de lumière différentes auxquelles est associée respectivement une unité de déflexion.

6. Microscope selon l'une des revendications précédentes, **caractérisé en ce qu'**il est prévu comme élément d'inversion au moins un agencement à micro-miroir qui est disposé directement dans le tube.

7. Microscope d'opération avec un microscope selon l'une des revendications précédentes.

8. , Procédé pour la superposition d'une première image vue à travers un objectif principal (8) d'un microscope (82) par au moins une deuxième image, **caractérisé en ce qu'**un rayon de lumière mince en faisceau (101) d'une source de lumière (64) est défléchi respectif modulé dans un dispositif de déflexion (102) et est introduit par un élément d'ouverture en fondu (32a) dans la marche des rayons (1) du microscope (82) de façon qu'il représente d'une manière visible la deuxième image pour l'oeil d'un observateur (100) **en ce que** le rayon de lumière (101) produit la deuxième image directement à la rétine de l'oeil de l'observateur (100).

9. Procédé selon la revendication 8, **caractérisé en ce que** le dispositif de déflexion (102) est commandé par les informations d'image obtenues à partir de l'objet (22) observé à travers le microscope (82) de telle sorte que, par exemple, des contours de détails d'objet (22a) sont redéfinis ou bien des détails d'objets (22a) sont représentés avec des lignes de réseau de grille ou analogue à l'emplacement effectif dans la zone du champ visible.

10. Procédé selon la revendication 9, **caractérisé en ce que** les informations d'image sont obtenues de préférence en trois dimensions à partir d'une installation de diagnostic de données (106) indépendantc du microscope, par exemple, une installation à rayons X, à CT-, à MRI-, à PE-, à ultrasons, où à la fois le microscope (82) et aussi l'installation de diagnostic de données (106), de préférence au moyen d'une mesure de test à un objet de test standard (109), est associé à un système de coordonnées commun (110).

11. Procédé selon la revendication 10, **caractérisé en ce que** les données d'image sont accordées au moyen d'une unité de correction les unes aux autres de façon à obtenir une coïncidence complète dans la position détaillée de l'objet.
